Europäisches Patentamt

European Patent Office

Office européen des brevets

(19)

(11) Numéro de publication: **0 083 285**
**A1**

(12) **DEMANDE DE BREVET EUROPEEN**

(21) Numéro de dépôt: **82402364.2**

(22) Date de dépôt: **22.12.82**

(51) Int. Cl.³: **A 61 K 31/235**
**A 61 K 35/10, A 61 K 9/06**
**//(A61K35/10, 31/235, 31/11),**
**(A61K35/10, 31/235, 31/19)**

(30) Priorité: **23.12.81 FR 8124065**

(43) Date de publication de la demande:
**06.07.83 Bulletin 83/27**

(84) Etats contractants désignés:
**BE CH DE GB IT LI NL**

(71) Demandeur: **Société LE THERMOGENE**
**176 rue de l'Arbrisseau**
**F-59000 Lille(FR)**

(72) Inventeur: **Werotte, Louis**
**77, avenue des Campanules**
**B-1170 Bruxelles(BE)**

(74) Mandataire: **Barbin le Bourhis, Joel**
**Cabinet M. SABATIER 83 Avenue Foch**
**F-75116 Paris(FR)**

(54) **Composition analgésique comprenant au moins un phtalate acide d'alcool neutralisé par une base.**

(57) Composition analgésique conditionnée sous forme d'onguent.

Selon l'invention, le principal constituant actif de la composition est un phtalate acide d'alcool neutralisé par une base, de préférence, le phtalate de buthyle et de sodium.

La composition peut en outre comporter un extrait de tourbe.

EP 0 083 285 A1

L'invention concerne une composition analgésique, notamment une crème, présentant une efficacité soutenue à l'égard de nombreuses formes de douleurs.

On connaît de nombreuses préparations topiques contre les douleurs (rhumatismes, contusions, etc) mettant en valeur les propriétés analgésiques reconnues d'un/ou plusieurs produits chimiques. La liste des substances naturelles ou de synthèse possèdant des propriétés analgésiques est certainement loin d'être close. La présente invention concrétise des recherches et des essais prolongés pour mettre en évidence et valoriser les propriétés analgésiques inconnues jusqu'à présent de certaines substances.

L'un des objets de l'invention est donc de mettre en valeur et permettre l'exploitation en tant qu'analgésiques de phtalate acides de certains alcools, neutralisés par une base.

Un autre objet de l'invention est d'incorporer ces substances dans une crème, de préférence avec addition d'autres produits déjà connus pour une certaine efficacité contre au moins certaines douleurs, notamment des substances pouvant être obtenues à partir d'extraits de produits naturels complexes comme la tourbe. Dans cet esprit, l'invention concerne donc une composition analgésique caractérisée en ce que l'un au moins de ses constituants actifs est un phtalate acide d'alcool neutralisé par une base, de préférence, le phtalate de n-butyle et de sodium, dans une proportion en poids comprise entre 0,1 et 5%, de préférence comprise entre 2 et 3%.

Une composition analgésique préférée comporte en outre au moins un second constituant actif choisi dans le groupe comprenant : la vanilline, l'acide vanillique, l'acétovanillone et l'acide férulique, dans la proportion en poids comprise entre 0,2 et 2,5%.

Une autre composition préférée est caractérisée en ce qu'elle comporte en outre, en tant que constituant actif, un extrait de tourbe, de préférence un extrait de tourbe de sphaigne.

Ce /ou ces constituants actifs peuvent être incorporés dans une crème de base comprenant de l'eau, au moins un alcool gras solide à température ambiante comme par exemple l'alcool cétylique et au moins un alcool gras sulfaté comme par exemple le laurylsulfate de sodium. Il est avantageux d'ajouter à cette crème de base un corps gras en tant qu'adjuvant permettant d'améliorer la pénétration dans la peau: l'huile de maïs donne de bons résultats. Il est également recommandé d'incorporer à la composition selon l'invention une faible proportion, de l'ordre de 0,05% d'un antiseptique tel que le parahydroxybenzoate de méthyle ou Nipagine en tant que conservateur et notamment antifongique. D'autres conservateurs sont également recommandés, comme le butylhydroxyanisol (BHA) utilisé dans une proportion comprise entre 0,01 et 0,1% en tant qu' antioxygène, ainsi que du citrate de sodium incorporé à raison de 0,1 à 0,5% en poids. Les proportions indiquées ci-dessus de citrate de sodium et de BHA permettent d'éviter la coloration au vieillissement de la crème, cette dernière étant normalement blanche lorsque l'extrait de tourbe mentionné plus haut n'y est pas incorporé. Enfin, on peut parfumer la préparation. On utilise de préférence à ce titre l'huile essentielle de lavande, incorporée à raison de 0,1 à 0,5% en poids.

On va maintenant indiquer un exemple actuellement préféré d'une composition analgésique selon l'invention :

| | |
|---|---|
| Alcool cétylique | 150 g |
| Laurylsulfate de sodium | 20 g |
| Huile de maïs | 25 g |
| Phtalate de n-butyle et de sodium | 25 g |
| Vanilline | 7,5 g |

| | |
|---|---|
| Butylhydroxyanisol (BHA) | 0,5 g. |
| Parahydroxybenzoate de méthyle | 0,5 g. |
| Citrate de sodium | 4 g |
| Huile essentielle de lavande | 2 g. |
| Eau | 765,5 g |

De façon connue, le crème de base est constituée par l'alcool cétylique dispersé dans l'eau au moyen du laurylsulfate de sodium. Les quantités respectives d'alcool cétylique, de laurylsulfate et d'eau peuvent être modifiées pour faire varier la consistance de la crème. Tout ou partie de l'alcool cétylique peut être remplacé par un autre alcool gras solide à température ambiante, par exemple l'alcool myristylique ou l'alcool stéarylique. Le laurylsulfate de sodium peut quant à lui être remplacé en tout ou partie par un autre alcool gras sulfaté tel que le stéarylsulfate de sodium.

Pour obtenir 25 gr de phtalate de -butyle et de sodium, on neutralise 22,75 gr de phtalate acide de n-butyle par 5,45 gr de carbonate anhydre de sodium, en présence d'une partie de l'eau nécessaire pour préparer la crème. Le phtalate acide est quant à lui préparé à partir de l'anhydride phtalique et de l'alcool n-butylique. On procède à la réaction de ces deux constituants en les mettant en présence et en les chauffant pendant deux heures au bain-marie. Le phtalate acide de butyle ainsi obtenu, accompagné d'un excès d'alcool est coulé dans un récipient dans lequel on le laisse se solidifier. On concasse ensuite ce produit et on l'étale pour permettre une évaporation de l'excès d'alcool. Le phtalate acide est ensuite neutralisé à la demande par du carbonate de sodium anhydre en présence d'eau chauffée aux environs de 60 à 65°. Il est préférable de préparer le phtalate de butyle et de sodium à la demande et dans l'eau servant à la préparation de la crème, à cause de sa très grande solubilité dans l'eau qui en rend la récupération difficile.

Comme mentionné précédemment, on peut utiliser un phtalate d'un autre alcool. On préfèrera la plupart du temps un alcool primaire pour des raisons de stabilité vis à vis de l'hydrolyse. Le phtalate acide de n-butyle a été retenu en raison de sa très faible toxicité. L'emploi du carbonate de sodium pour neutraliser le phtalate acide n'est pas non plus limitatif et toute base pharmaceutiquement acceptable peut être utilisée.

Selon une variante de la composition analgésique indiquée ci-dessus, on peut remplacer l'eau par un extrait de tourbe de sphaigne dont le procédé de préparation est le suivant :

On chauffe un mélange de tourbe et d'eau aux environs de 50 à 60° et on ajoute une quantité de carbonate de sodium anhydre jusqu'à faire remonter le pH aux environs de 7,2 à 7,5. On laisse ensuite la réaction s'effectuer pendant environ 15 à 20 minutes en ramenant éventuellement de temps en temps le pH à la valeur indiquée ci-dessus. La préparation est ensuite tamisée et on laisse décanter l'extrait ainsi obtenu pendant environ 48 heures avant d'effectuer un dernier filtrage.

La combinaison dans une même crème des trois constituants actifs mentionnés ci-dessus (phtalate, vanilline et extrait de tourbe) permet d'élargir le champs d'application de la crème selon l'invention, avec une réaction positive ou très positive sur la quasi totalité des patients. La composition selon l'invention peut être utilisée avec succès pour lutter contre des douleurs provenant d'origines aussi diverses que les rhumatismes, la fatigue musculaire, les contusions, les abcès dentaires, la migraine, les mycoses et la sinusite.

Bien entendu, l'invention n'est pas limitée à la composition indiquée ci-dessus mais comprend toutes les compositions équivalentes définies par les revendications qui suivent.

REVENDICATIONS

1. Composition analgésique caractérisée en ce que l'un au moins de ses constituants actifs est un phtalate acide d'alcool neutralisé par une base, de préférence le phtalate de n-butyle et de sodium, dans une proportion en poids comprise entre 0,1 à 5%, de préférence comprise entre 2 et 3%.

2. Composition selon la revendication 1, caractérisée en ce que la proportion dudit phtalate acide d'alcool neutralisé est voisine de 2,5%.

3. Composition selon la revendication 1 ou 2, caractérisée en ce qu'elle comporte en outre au moins un second constituant actif choisi dans le groupe comprenant : la vanilline, l'acide vanillique, l'acétovanillone et l'acide férulique dans une proportion en poids comprise entre 0,2 et 2,5%, de préférence comprise entre 0,5 et 1%.

4. Composition selon la revendication 3, caractérisée en ce que la proportion en poids dudit second constituant actif, de préférence la vanilline, est voisine de 0,75%.

5. Composition selon l'une des revendications précédentes, caractérisée en ce qu'elle comporte en outre, en tant que constituant actif, un extrait de tourbe, de préférence un extrait de tourbe de sphaigne.

6. Composition selon l'une des revendications précédentes, caractérisée en ce que le/ou les constituants actifs précités sont incorporés dans une crème de base comprenant de l'eau, au moins un alcool gras solide à température ambiante et au moins un alcool gras sulfaté.

7. Composition selon la revendication 6, caractérisée en ce que ledit alcool gras solide à température ambiante est l'alcool cétylique et ledit alcool gras sulfaté est le laurylsulfate de sodium.

8. Composition selon la revendication 6 ou 7, caractérisée en ce que ledit alcool gras solide à température ambiante est présent dans la proportion en poids d'environ 15% et ledit alcool gras sulfaté est présent dans la proportion en poids d'environ 2%.

9. Composition selon l'une des revendications 6 à 8, caractérisée en ce que ladite crème de base comporte en outre un corps gras en tant qu'adjuvant permettant d'améliorer la pénétration dans la peau, par exemple l'huile de maïs dans la proportion en poids d'environ 2,5%.

10. Composition selon l'une des revendications précédentes, caractérisée en ce qu'elle contient une proportion d'au moins 0,05% de parahydroxybenzoate de méthyle, notamment en tant qu'agent antiseptique.

11. Composition selon l'une des revendications précédentes, caractérisée en ce qu'elle contient une proportion de butylhydroxyanisol comprise entre 0,01 et 0,1% en poids, de préférence 0,05%, notamment en tant qu'antioxygène.

12. Composition selon l'une des revendications précédentes, caractérisée en ce qu'elle comprend une proportion de citrate de sodium comprise entre 0,1 et 0,5% en poids, de préférence 0,4%, notamment en tant que conservateur.

13. Composition selon l'une des revendications précédentes, caractérisée en ce qu'elle contient une proportion d'huile essentielle de lavande comprise entre 0,1 et 0,5% en poids, de préférence 0,2%, notamment en tant que parfum.

14. Composition selon la revendication 5, caractérisée en ce que l'extrait de tourbe précité est essentiellement le résultat d'une réaction provoquée entre de la tourbe et du carbonate de sodium anhydre, en présence d'eau.

**Office européen des brevets**

## RAPPORT DE RECHERCHE EUROPEENNE

EP 82 40 2364

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-A-2 138 929 (IMPERIAL CHEMICAL IND. LTD.)  --- | 1-14 | A 61 K 31/235<br>A 61 K 35/10<br>A 61 K 9/06 //<br>(A 61 K 35/10 |
| A | FR-M- 7 919 (BOOTS PURE DRUG COMP. LTD.)  ----- | 1-14 | A 61 K 31/235<br>A 61 K 31/11 )<br>(A 61 K 35/10<br>A 61 K 31/235<br>A 61 K 31/19 ) |

**DOMAINES TECHNIQUES RECHERCHES (Int. Cl. ³)**

A 61 K
C 07 C

Le présent rapport de recherche a été établi pour toutes les revendications

| Lieu de la recherche<br>LA HAYE | Date d'achèvement de la recherche<br>24-03-1983 | Examinateur<br>BRINKMANN C. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-ecrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82